Europäisches Patentamt

European Patent Office

Office européen des brevets

(11) Veröffentlichungsnummer: **0 496 196 A1**

# (12) EUROPÄISCHE PATENTANMELDUNG

(21) Anmeldenummer: **92100086.5**

(22) Anmeldetag: **04.01.92**

(51) Int. Cl.5: **A61M 21/00**, G04C 23/00, A61B 5/16

(30) Priorität: **19.01.91 DE 4101471**

(43) Veröffentlichungstag der Anmeldung:
**29.07.92 Patentblatt 92/31**

(84) Benannte Vertragsstaaten:
**AT BE CH DE DK ES FR GB GR IT LI NL PT SE**

(71) Anmelder: **Schroeder, Dirk**
**Morgartenstrasse 30**
**W-4600 Dortmund 1(DE)**

(72) Erfinder: **Schroeder, Dirk**
**Morgartenstrasse 30**
**W-4600 Dortmund 1(DE)**

(74) Vertreter: **Patentanwälte Meinke und**
**Dabringhaus Dipl.-Ing. J. Meinke Dipl.-Ing. W.**
**Dabringhaus**
**Westenhellweg 67**
**W-4600 Dortmund 1(DE)**

(54) **Verfahren und Vorrichtung zur Steuerung von elektronischen Einrichtungen durch die unterschiedlichen Schlafphasen eines Menschen.**

(57) Mit einem Verfahren und einer Vorrichtung zur Nutzung der unterschiedlichen Wach-/Schlafphasen eines Menschen zur Betätigung bzw. Steuerung von insbesondere elektronischen Einrichtungen, soll eine Lösung geschaffen werden, mit der der Verlauf von Wachen und Schlafen einer Person gezielt zur Beeinflussung von Einrichtungen herangezogen werden kann, mit der insbesondere die jeweils aktuelle und/oder vorausberechenbare Wach-/Schlafphase einer zu weckenden Person mit dem Weckverhalten eines gekoppelten Weckers koordiniert werden kann.

Dies wird dadurch erreicht, daß einem Steuergerät mit einem Datenverarbeitungsprogramm personenbezogene Daten, körperbezogene Daten, Zeitintervalle, Zeitabstände und/oder Festzeiten eingegeben und direkt oder über das Datenverarbeitungsprogramm Übertragungs-, Steuerungs-, Schalt- und/oder Auslöseimpulse bewirkt werden.

EP 0 496 196 A1

Die Erfindung richtet sich auf ein Verfahren zur Nutzung der unterschiedlichen Wach-/Schlafphasen eines Menschen zur Betätigung bzw. Steuerung von z.B. elektronischen Einrichtungen sowie auf eine entsprechende Vorrichtung.

Es ist bekannt, daß die Schlaftiefe eines Menschen über den Schlafzeitraum gesehen nicht gleichmäßig über die Zeit ausgebildet ist. Es gibt Phasen des Tiefschlafes, des sogenannten Flachschlafes und Zwischenphasen. Diese unterschiedliche Schlafintensität kann auf verschiedene Weise gemessen werden, hier sei lediglich als Beispiel die Gehirnstrommessung genannt, die Messung der Körpertemperatur, die Messung der Motorik während des Schlafens u. dgl. mehr.

Aufgabe der Erfindung ist die Schaffung einer Lösung, mit der der Verlauf von Wachen und Schlafen einer Person gezielt zur Beeinflussung von Einrichtungen herangezogen werden kann, mit der insbesondere die jeweils aktuelle und/oder vorausberechenbare Wach-/Schlafphase einer zu weckenden Person mit dem Weckverhalten eines gekoppelten Weckers koordiniert werden kann.

Mit einem Verfahren der eingangs bezeichneten Art wird diese Aufgabe gemäß der Erfindung dadurch gelöst, daß einem Steuergerät mit einem Datenverarbeitungsprogramm personenbezogene Daten, körperbezogene Daten, Zeitintervalle, Zeitabstände und/oder Festzeiten eingegeben und direkt oder über das Datenverarbeitungsprogramm Schalt- und Auslöseimpulse bewirkt werden.

Mit einer derartigen Verfahrensweise ist es möglich, etwa während des Schlafens bestimmte Schlafphasen auszunutzen, um ein Lernprogramm der schlafenden Person bereitzustellen. Damit läßt sich erreichen, daß während des Schlafens gelernt werden kann, um etwa die hohe Aufnahmefähigkeit des Gehirns während einer Traumphase zum Lernen zu nutzen. Diese Phasen können mehrfach hintereinander abgerufen werden. Durch die Überwachung der Schlaftiefenkurve des Menschen können auch etwa bei speziellen Krankheitssymptomen Schlafphasen durch Musikhinterlegung intensiviert werden, um einen Heilungsprozeß zu beschleunigen.

Ein besonderes Einsatzfeld ist dabei die Optimierung der Beendigung einer Schlafphase durch das Wecken der entsprechenden Person. Hierzu sieht die Erfindung in Ausgestaltung vor, daß körperbezogene Daten, wie die Körpertemperatur und/oder die Körperbewegungen des Schläfers und/oder die Gehirnstromkurve (EEG), über die Schlafzeit gemessen und daraus eine Schlaftiefenkurve ermittelt und der Weckzeitpunkt in eine wählbare Schlafphase, z.B. einem Flachschlafbereich, positioniert wird.

Durch diese erfindungsgemäße Verfahrensweise ist es für den Benutzer möglich, dem Wecker einen Spielraum für das Auslösen des Weckalarmes zu geben, wobei der Wecker über ein Datenverarbeitungsprogramm personenbezogene Daten erfassen und auswerten kann, um innerhalb des Weckintervalles den Weckalarm optimal auszulösen. Mit personenbezogenen Daten sind solche Daten gemeint, die aktuellen Schwankungen nicht unterliegen, etwa Geburtstag, Geburtsort, Geschlecht u. dgl.

Es sind Uhren bekannt, die neben der reinen Uhrenfunktion, so z.B. auch das automatische Umstellen von Sommer- auf Winterzeit, weitere Funktionen wahrnehmen können, etwa einen zusätzlichen Rechner aufweisen oder auch ein zusätzliches Programm zur Ermittlung eines Bio-Rhythmus, indem man das jeweilige Geburtsdatum eingibt. Eine solche Uhr stellt beispielsweise die Uhr "Casio BQ-1100" mit ihrer entsprechenden Bedienungsanleitung dar. Danach ein Wecksignal auszurichten, ist mit dieser Uhr nicht möglich.

In Ausgestaltung ist vorgesehen, daß körperbezogene Daten, wie die Körpertemperatur und/oder die Körperbewegungen des Schläfers und/oder die Gehirnstromkurve (EEG), über die Schlafzeit hinweg gemessen und daraus eine Schlaftiefenkurve ermittelt und der Weckzeitpunkt in einen Flachschlafbereich positioniert wird.

Durch diese Verfahrensweise wird erreicht, daß die zu weckende Person durch das Wecksignal beispielsweise nicht erschreckt, weil sie sich gerade in einer Tiefschlafphase befindet, woraus sich unmittelbar eine bessere Laune nach dem Weckzeitpunkt bei der geweckten Person ergibt. Die Stimmung ist besser, die zu absolvierenden Beschäftigungen nach dem Wecken fallen leichter.

Die Erfindung sieht auch vor, daß neben den personen- und/oder körperbezogenen Daten vom Benutzer programmierbare zusätzliche Daten zur Ermittlung eines optimalen Weckzeitpunktes dem Wecker zugeführt und/oder zur Bestimmung eines optimalen Weckzeitpunktes unter Berücksichtigung der eingegebenen Daten vom Benutzer dem Wecker eine maximale Weckzeitspanne eingegeben wird.

Zur Klarstellung sei angegeben, daß es bei jedem Menschen personenenbezogene Daten gibt, etwa das Geburtsdatum, der Geburtsort, ggf. das Geschlecht und andere, biologischen Schwankungen nicht unterworfene Daten. Darüber hinaus gibt es auch körperbezogene Daten, die den jeweils aktuellen körperlichen Zustand beschreiben, wie Körpertemperatur, Herz- und Gehirnrhythmus u. dgl. Schließlich gibt es weitere weder personennoch körperbezogene Daten, die allerdings auf den Weckzeitpunkt erhebliche Einflüsse haben können, etwa ob es sich um einen Arbeitstag oder um einen Feiertag handelt, Änderungen von Sonnenaufgang und Sonnenuntergang u. dgl. mehr.

Mit der Erfindung ist es darüber hinaus möglich, weitere Informationsquellen für den Wecker zur Verfügung zu stellen, etwa den Verkehrsfunk in das Weckprogramm mit einzuschließen, etwa über Btx-Informationen. So kann eine Stauprognose im morgendlichen Berufsverkehr vom Wecker über Datenfernübertragung erfaßt werden, wodurch aufgrund des Programmes ein frühzeitigeres Wecken erfolgt als in einer Situation, bei der dem Wecker derartige Informationen nicht vorliegen. Der Terminkalender kann mit einprogrammiert werden oder aber auch der Zweitwecker für einen Partner, wobei ggf. auch die personenbezogenen Daten des schlafenden Partners mit in die Steuerung des Weckzeitpunktes beider Personen einbezogen werden können.

In weiterer Ausgestaltung ist nach der Erfindung vorgesehen, daß nach Ermittlung des optimalen Weckzeitpunktes in ihrer Intensität sich ändernde Wecksignale optischer, akustischer und/oder mechanischer Art ausgelöst werden. Mit dieser Verfahrensweise kann erreicht werden, daß zum Wecken etwa zunächst akustische Signale sanfter Art ausgelöst werden, danach optische Signale, etwa durch Einschaltung der Raumbeleuchtung, wobei ggf. auch als mechanische Hilfsmittel die Schräglage des Schlafenden durch ein entsprechend steuerbares Bett verändert werden kann.

Zur Lösung der gestellten Aufgabe sieht die Erfindung auch eine Vorrichtung mit einem elektronisch beeinflußbaren Zeitmeßgerät mit Weckeinrichtung vor, die sich durch Signaleingänge zur Aufnahme von benutzerspezifischen personen- und/oder körperbezogene Daten, wie Körpertemperatur, Körperbewegungen, ggf. Gehirnströmen, und einer elektronischen Einrichtung zur Auswertung dieser Daten zur Erzeugung einer Schlaftiefenkurve und einer elektronischen Auswahleinrichtung zur Ermittlung eines Steuer- und/oder Weckzeitpunktes aufgrund eingegebener fester und ermittelter, sich ändernder aktueller Daten, auszeichnet.

Erkennbar ist es mit einer solchen Vorrichtung möglich, die Schlaftiefe zu berücksichtigen und z.B. bei einer möglichst geringen Schlaftiefe den Weckalarm auszulösen unter Berücksichtigung beispielsweise der Daten eines Terminkalenders, der vorgegebenen Zeiten der Morgentoilette und das Frühstücken unter Berücksichtigung von Wegezeiten zur Arbeitsstätte u. dgl. mehr. Mit der Erfindung können z.B. auch fruchtbare und unfruchtbare Tage einer Frau ermittelt und kenntlich gemacht werden.

Soweit hier von elektronischen Geräten, elektronischer Datenübertragung, elektronischer Datenerfassung u. dgl. die Rede ist, sei bemerkt, daß es sich hierbei auch um andere Arten der Steuerung, Datenerfassung u. dgl. handeln kann, etwa um hydraulische, pneumatische, elektromagnetische und bei der Datenverarbeitung und -erfassung insbesondere auch um bionische Verfahren und Vorrichtungen.

Weitere Ausgestaltungen der Vorrichtung ergeben sich aus den Unteransprüchen, wobei vorteilhafterweise eine Datenübernahmeschnittstelle vorgesehen sein kann zur Übernahme beispielsweise eines Notebook-PC-Rechners zur Berücksichtigung von Terminänderungen, -verschiebungen oder Wegfall von Terminen u. dgl. mehr. Genormte Schnittstellen machen es möglich, die Vorrichtung auf Reisen mitsichzuführen und Ergänzungseinrichtungen vor Ort zu nutzen.

Werden alle wesentlichen Schnittstellen oder Dateneingabespeicherungen u. dgl. erfindungsgemäß ausgenutzt, so können neben den personenbezogenen und oben bereits genannten sonstigen Informationen und Daten auch andere weckrelevante Daten mit einbezogen werden, etwa ein Einbruchalarm, Telefonanrufe, die über ISDN-Identifizierung des Anrufers gefiltert sein können, Mondphasen, Sonnenaufgangsphasen, Wetterdaten (beispielsweise Glatteis), Hochwasser, Defekte, Rauchmelder, ggf. auch Börsenveränderungen an ausländischen Börsenplätzen od. dgl., ermittelt werden, wobei als personenbezogene Daten bei weiblichen Benutzern auch die Monatszyklen eingegeben werden können.

Weitere Möglichkeiten: Errechnung des wahrscheinlich optimalen Einschlafzeitpunktes (Erfahrungsrückrechnung), Speicherung von verschiedenen Weckprogrammen, die langzeitterminkalender-gesteuert sein können und automatisch z.B. Ausschlafmöglichkeiten an Feiertagen, Wochenenden etc. berücksichtigen. Die Weckart (Lautstärke, Musik, individuelle Ansprache, Fenster-/Rollladenöffnung), Art des Frühstücksgetränkes, die Stärke des Kaffees in der automatisch eingeschalteten Kaffeemaschine kann von der Schlafart (ruhig/unruhig) und Schlaflänge abhängig gemacht werden. Durch das Weckprogramm kann auch im Vorgriff die Heizung eines geparkten Fahrzeuges eingeschaltet werden.

Möglich ist auch die Beachtung von Daten des Gesundheitsbildes (Fieber, Schweiß, Restalkohol) und z.B. die Ansage "Sie haben 39°C Fieber". Bei unruhigem, schlechten Schlaf können beruhigende Geräusche (sound-soother) zugeschaltet werden, die beruhigend wirken. Im Extremfall können beunruhigende Herzrhythmusveränderungen oder gar Herzstillstand erkannt und ggf. z.B. telefonisch gemeldet werden.

Zweckmäßig trägt eine die Vorrichtung benutzende Person ein Datenübertragungselement etwa in Art einer Armbanduhr mit Datensensoren zur Aufnahme von körperspezifischen Daten und einem Sender zur Übermittlung dieser Daten an die erfindungsgemäße Vorrichtung, wobei die Erfindung auf diese spezielle Ausgestaltung der Datenaufnahme und -übertragung nicht beschränkt ist. So kann beispielsweise die Körpertemperatur über Sensoren in der Bettdecke aufgenommen werden u. dgl. mehr.

Eine besondere Möglichkeit, Informationen über das Schlafverhalten einer Person zu bekommen, ist deren Motorik während des Schlafens. So können durch einfache Bewegungssensoren Bewegungen einer schlafenden Person unmittelbar am Bettgestell gemessen werden. Über entsprechend sensible Magnetfeldmessungen können auch die körpereigenen Magnetfelder bzw. deren Schwankungen erfaßt werden. Neben der Körperbewegung als solche kann auch alleine die Augenbewegung überwacht werden u. dgl. mehr.

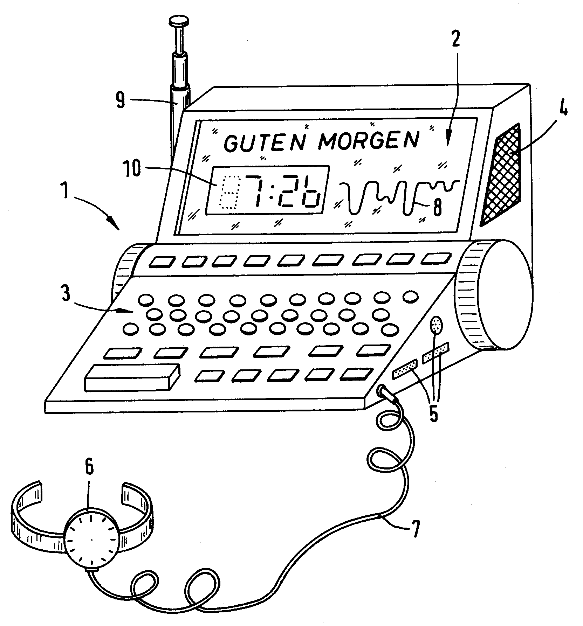

Die Erfindung ist nachstehend anhand der Zeichnung beispielsweise näher erläutert. Diese zeigt in stark vereinfachter Darstellung eine Vorrichtung nach der Erfindung.

Die allgemein mit 1 bezeichnete Vorrichtung weist im dargestellten Beispiel, ähnlich wie ein sogenanntes Lap-Top, ein Anzeigenfeld 2 auf zur Anzeige von Text, Uhrzeit u. dgl., ein Tastenfeld 3 mit Programmiertasten und ggf. Tasten zum Einstellen eines Radios od. dgl., wobei hier andeutungsweise ein Lautsprecherfeld 4 dargestellt ist.

Wesentlich für die Erfindung sind neben den üblichen Bedienungseinheiten Schnittstellen 5 zum Anschließen von Signaleingängen etwa zur Aufnahme von benutzerspezifischen Daten, wie der Körpertemperatur, der Körperbewegung, der Gehirnströme u. dgl. Um die Aufnahme derartiger Daten zu symbolisieren ist hier eine Datenaufnahmeeinrichtung 6 mit einem Anschlußkabel 7 dargestellt, die beispielsweise um das Handgelenk eines Benutzers gelegt werden kann.

Über diese Aufnahmeeinrichtung 6 können die oben genannten benutzerspezifischen Daten abgefragt werden. So kann dort ein Temperatursensor vorgesehen sein, ebenso wie sonstige andere Aufnahmeelemente etwa zur Messung des Pulses, des Hautwiderstandes, der Hautoberflächenfeuchtigkeit u. dgl. mehr.

Nicht näher dargestellt ist in der Zeichnung die elektronische Ausstattung der Vorrichtung 1 mit Mikroprozessoren od. dgl., da diese in unterschiedlichen Varianten durchführbar sind. Wesentlich ist, daß eine sogenannte Wach-/Schlaftiefenkurve ermittelbar und auswertbar ist. Eine solche Wach-/Schlaftiefenkurve mit unterschiedlichen Wach-, Tief- und Flachschlafphasen ist auf dem Anzeigefeld 2 angedeutet und mit 8 bezeichnet.

Die Vorrichtung ist auch zur Datenfernübertragung ausgerüstet, dies ist nur symbolisch durch eine Antenne 9 angedeutet.

Natürlich ist die Erfindung nicht auf das beschriebene Ausführungsbeispiel beschränkt. So kann die lediglich auf dem Anzeigefeld 2 angedeutete Uhr 10 auch durch eine eigene digitale oder analoge Anzeige ersetzt sein, es können optische und akustische Signale vorgesehen sein u. dgl. mehr. Die Vorrichtung kann schließlich auch eingesetzt werden, um z.B. das Einschlafen einer einschlafgefährdeten Person (z.B. Fernfahrer) zu überwachen und zu verhindern.

**Patentansprüche**

1. Verfahren zur Nutzung der unterschiedlichen Wach-/Schlafphasen eines Menschen zur Betätigung bzw. Steuerung von insbesondere elektronischen Einrichtungen,
dadurch gekennzeichnet,
daß einem Steuergerät mit einem Datenverarbeitungsprogramm personenbezogene Daten, körperbezogene Daten, Zeitintervalle, Zeitabstände und/oder Festzeiten eingegeben und direkt oder über das Datenverarbeitungsprogramm Übertragungs-, Steuerungs-, Schalt- und/oder Auslöseimpulse bewirkt werden.

2. Verfahren nach Anspruch 1 zur Beendigung der Schlafphase eines Menschen,
dadurch gekennzeichnet,
daß körperbezogene Daten, wie die Körpertemperatur und/oder die Körperbewegungen des Schläfers und/oder die Gehirnstromkurve (EEG), über die Schlafzeit gemessen und daraus eine Wach-/Schlaftiefenkurve ermittelt und der Weckzeitpunkt in eine wählbare Schlafphase, z.B. einem Flachschlafbereich, positioniert wird.

3. Verfahren nach Anspruch 2,
dadurch gekennzeichnet,
daß neben den personen- und/oder körperbezogenen Daten vom Benutzer programmierbare zusätzliche Daten zur Ermittlung eines optimalen Weckzeitpunktes dem Wecker zugeführt werden.

4. Verfahren nach einem der vorangehenden Ansprüche,
dadurch gekennzeichnet,
daß nach Ermittlung des optimalen Weckzeitpunktes in ihrer Intensität sich ändernde Wecksignale optischer, akustischer und/oder mechanischer Art ausgelöst werden.

5. Vorrichtung insbesondere zur Durchführung des Verfahrens nach einem der vorangehenden Ansprüche, mit einem elektronisch beeinflußbaren Zeitmeßgerät mit Weckeinrichtung,
gekennzeichnet durch
Signaleingänge (5) zur Aufnahme von benutzerspezifischen Daten, wie Körpertemperatur, Körperbewegungen, ggf. Gehirnströmen, und einer elektronischen Einrichtung zur Auswertung dieser Daten zur Erzeugung einer Wach-/Schlaftiefenkurve (8) und einer z.B. elektronischen Auswahleinrichtung zur Ermittlung eines Steuer- und/oder Weckzeitpunktes aufgrund eingegebener und ermittelter aktueller Daten.

6. Vorrichtung nach Anspruch 5,
gekennzeichnet durch
zusätzliche Datenverarbeitungseinrichtungen zur Übernahme externer Daten, wie der an sich bekannten funkferngesteuerten Weltzeituhr (10), des Verkehrsfunkes oder Weckdaten eines Schlafpartners.

7. Vorrichtung nach Anspruch 5 oder 6,
gekennzeichnet durch
eine Datenübernahmeschnittstelle (5) zur Übernahme von externen Daten, wie z.B. den Daten eines Terminkalenders.

8. Vorrichtung nach Anspruch 5 oder einem der folgenden,
gekennzeichnet durch
eine Datenausgabeeinrichtung zur Erzeugung von unterschiedlichen Weckarten über akustische, optische oder sonstige elektrische oder elektronische Impulse.

9. Vorrichtung nach Anspruch 5 oder einem der folgenden,
gekennzeichnet durch
eine ggf. drahtlose Fernübertragung (6) mit Datensensoren und Datensendern z.B. am Körper des Benutzers und einem Datenempfänger an der Vorrichtung (1).

GUTEN MORGEN

7:26

# EUROPÄISCHER RECHERCHENBERICHT

## EINSCHLÄGIGE DOKUMENTE

EP 92100086.5

| Kategorie | Kennzeichnung des Dokuments mit Angabe, soweit erforderlich, der maßgeblichen Teile | Betrifft Anspruch | KLASSIFIKATION DER ANMELDUNG (Int Cl.⁵) |
|---|---|---|---|
| X | GB - A - 2 233 764 (MATSUSHITA) | 1,2,4, 5,8,9 | A 61 M 21/00 G 04 C 23/00 A 61 B 5/16 |
| Y | * Zusammenfassung; Fig. 1; Seite 1, Zeile 1 - Seite 6, Zeile 23; Seite 10, Zeile 24 - Seite 18, Zeile 12 * | 6,7 | |
| X | US - A - 4 228 806 (LIDOW) | 1,2,5 | |
| Y | * Zusammenfassung; Fig. 3,4; Spalte 1, Zeilen 1-47; Spalte 2 ganz; Spalte 3, Zeilen 1-32; Ansprüche * | 6,7 | |
| Y | ELEKTOR, Band 9, Nr. 129, September 1981, Elektor Verlag, Gangelt, "DCF Computer-Schaltuhr", Seiten 9-58 - 9-67 * Seiten 9-58 * | 6 | |
| X | DD - A - 263 238 (BEZIRKSNERVENKLINIK SCHWERIN) * Gesamt * | 1,2,4 | RECHERCHIERTE SACHGEBIETE (Int Cl.⁵) A 61 B A 61 M G 04 B |
| Y | US - A - 4 162 610 (LEVINE) | 7 | |
| A | * Zusammenfassung; Spalte 1, Zeilen 26-68; Ansprüche * | 3 | |
| A | DE - A - 3 102 239 (KARRAS) * Zusammenfassung; Anspruch; Seite 4, Zeile 10-Ende * | 1,2 | |
| | DE - A - 2 405 771 | 4 | |

Der vorliegende Recherchenbericht wurde für alle Patentansprüche erstellt.

| Recherchenort | Abschlußdatum der Recherche | Prüfer |
|---|---|---|
| WIEN | 27-03-1992 | ZAWODSKY |

| Kategorie | Kennzeichnung des Dokuments mit Angabe, soweit erforderlich, der maßgeblichen Teile | Betrifft Anspruch | KLASSIFIKATION DER ANMELDUNG (Int. Cl⁵) |
|---|---|---|---|
| | (GFU) <br> * Seiten 1-3, Zeile 11 * <br> ---- | | |

**EINSCHLÄGIGE DOKUMENTE**

-2-
EP 92100086.5

RECHERCHIERTE SACHGEBIETE (Int. Cl⁵)

Der vorliegende Recherchenbericht wurde für alle Patentansprüche erstellt.

| Recherchenort | Abschlußdatum der Recherche | Prüfer |
|---|---|---|
| WIEN | 27-03-1992 | ZAWODSKY |